# EUROPEAN PATENT APPLICATION

(11) **EP 4 846 972 A1**
(43) Date of publication of application: **02.12.2026**
(21) Application number: 25744887.8
(22) Date of filing: 14.01.2025
(51) Int. Cl.: A61B 34/37, B25J 3/00

(54) **MASTER/SLAVE SYSTEM, SYNCHRONOUS SWITCHING CONTROL METHOD, AND PROGRAM**

(30) Priority: 26.01.2024 JP 2024010035
(71) Applicant: Qoly Corporation, Tokyo 141-0032 (JP)
(72) Inventor: OGIWARA, Kohei, Tokyo 108-0075 (JP); WAKANA, Kazuhito, Tokyo 108-0075 (JP); NAGAO, Daisuke, Tokyo 108-0075 (JP)
(74) Representative: SSM Sandmair
(86) International application number: PCT/JP2025/000851
(87) International publication number: WO 2025/158956

(57) **Abstract**

A master-slave system of the present disclosure includes a determination unit that determines, in operation of a master robot and a slave robot, whether or not an operation interface of the master robot that is operated by a user is deviated from a predetermined operation area, and a synchronous switching unit that performs switching so as to disable the synchronization between the slave robot and the master robot when the operation interface is deviated from the predetermined operation area.

## Description

### Field

The present disclosure is related to a master-slave system, a synchronous switching control method, and a program.

### Background

A master-slave system that includes a master and a slave (hereinafter, also described as a "robot arm") is known. The master includes an input device that allows an operator to operate the robot arm. The slave includes a robot hand (hereinafter, also described as an "end effector") corresponding to a target task. In the master-slave system, the slave performs a motion of a user (operator) that is input from the master, at actual size or scaled.

In the master-slave system, operation of switching between a synchronous state and an asynchronous state between the master and the slave, such as disengagement of a clutch to change a tool at an end of the robot arm depending on a task to be executed, frequently occurs.

In the operation of switching between synchronous and asynchronous, such as clutch operation, operation on the master occurs in the asynchronous state, and therefore, deviation of positions or postures (hereinafter, also collectively described as "positional deviation") occurs between the master and the slave. The positional deviation in the master-slave system is a factor that reduces operability, and therefore, it is needed to correct the positional deviation.

Conventionally, a method that allows a user to adjust a position and a posture of the master with respect to a position and a posture of the slave before switching between synchronous and asynchronous is known. In this method, the user operates the master by referring to the position and the posture of the slave that is displayed on a screen, and adjusts the position and the posture of the slave to eliminate the positional deviation that has occurred in the asynchronous state.

### Citation List

### Patent Literature

Patent Literature 1: Japanese Laid-open Patent Publication No. 2008-228967

### Summary

### Technical Problem

In the technology described in Patent Literature 1, to correct the positional deviation between the master and the slave, the position and the posture of the slave are output to a video output device, such as a monitor. Further, the user operates the master to perform positional alignment with the slave with reference to the position and the posture of the slave that are output by the video output device. In the method described in Patent Literature 1, the master does not operate autonomously, and therefore, it is not needed to mount an actuator for autonomous operation.

However, even when it is not needed to mount the actuator, the operation of switching between synchronous and asynchronous, such as the clutch operation, frequently occurs in a master-slave robot system, so that it is expected that the operation becomes more complicated.

The present disclosure has been conceived in view of the foregoing situation, and an object of the present disclosure is to eliminate positional deviation that frequently occurs between a master and a slave in a master-slave system and improve operability of the master-slave system.

### Solution to Problem

A master-slave system of the present disclosure includes a determination unit that determines, in operation of a master robot and a slave robot, whether or not an operation interface of the master robot that is operated by a user is deviated from a predetermined operation area, and a synchronous switching unit that performs switching so as to disable the synchronization between the slave robot and the master robot when the operation interface is deviated from the predetermined operation area.

### Brief Description of Drawings

FIG. 1 is a diagram illustrating a configuration of a master-slave system according to a first embodiment.
FIG. 2 is a diagram illustrating configuration examples of a master device and a slave device according to the first embodiment.
FIG. 3 is a side view for explaining a slave-side operation area and a slave-side safe area.
FIG. 4 is a diagram for explaining a definition of a slave-side safe area in a robot model.
FIG. 5 is a diagram illustrating configuration examples of master input devices on the left and right of a master according to the first embodiment.
FIG. 6 is a diagram illustrating the master input devices on the left and right of the master at the time of user operation according to the first embodiment.
FIG. 7 is a diagram illustrating states of the master input devices on the left and right of the master at the time of trigger execution according to the first embodiment.
FIG. 8 is a top view of a holder and a master operation unit when a holder-type trigger according to the first embodiment is used.
FIG. 9 is a side view of the holder and the master operation unit when the holder-type trigger according to the first embodiment is used.
FIG. 10 is a flowchart for explaining operation of the master-slave system according to the first embodiment.
FIG. 11 is a diagram illustrating an operation area and a safe area of a robot.
FIG. 12 is a diagram illustrating a state in which a right-side holder of the master is automatically moved.
FIG. 13 is a diagram illustrating an example of calibration of a safe area according to the first embodiment.
FIG. 14 is a diagram illustrating configuration examples of a left-side master input device and a right-side master input device of a master-slave system according to a second embodiment.
FIG. 15 is a diagram illustrating a state in which the right-side master input device of the master-slave system according to the second embodiment executes a trigger.
FIG. 16 is a flowchart for explaining operation of the master-slave system according to the second embodiment.
FIG. 17 is a hardware configuration diagram illustrating an example of a computer that implements an arithmetic device of a master-slave system 1 as an information processing apparatus according to the first embodiment and the second embodiment.

### Description of Embodiments

Preferred embodiments of the present disclosure will be described in detail below with reference to the accompanying drawings. In the present specification and the drawings, components that have substantially the same functional configurations are denoted by the same reference symbols, and repeated explanation will be omitted. Explanation will be given in the following order.
1. Embodiments
   1-1. First embodiment
      1-1-1. Definition of slave-side operation area and slave-side safe area
      1-1-2. Operation interface of master
      1-1-3. Relationship between holder and master operation unit
      1-1-4. Operation
      1-1-5. Example of calibration method
      1-1-6. Effects of first embodiment
   1-2. Second embodiment
      1-2-1. Operation
      1-2-2. Effects of second embodiment
2. Other embodiments
3. Effects
4. Hardware configuration
5. Notes

### 1. Embodiments

### 1-1. First embodiment

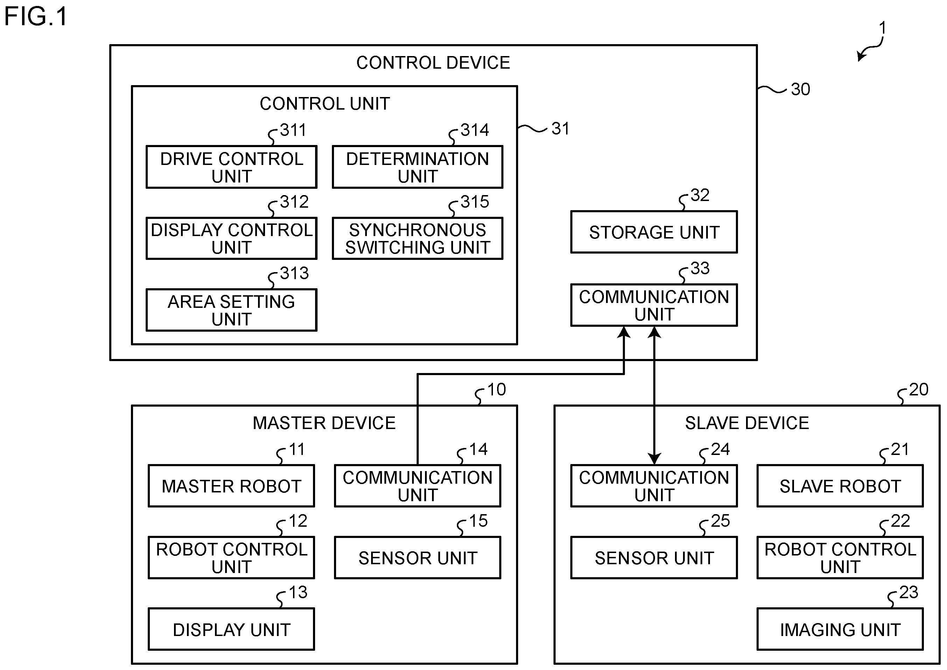

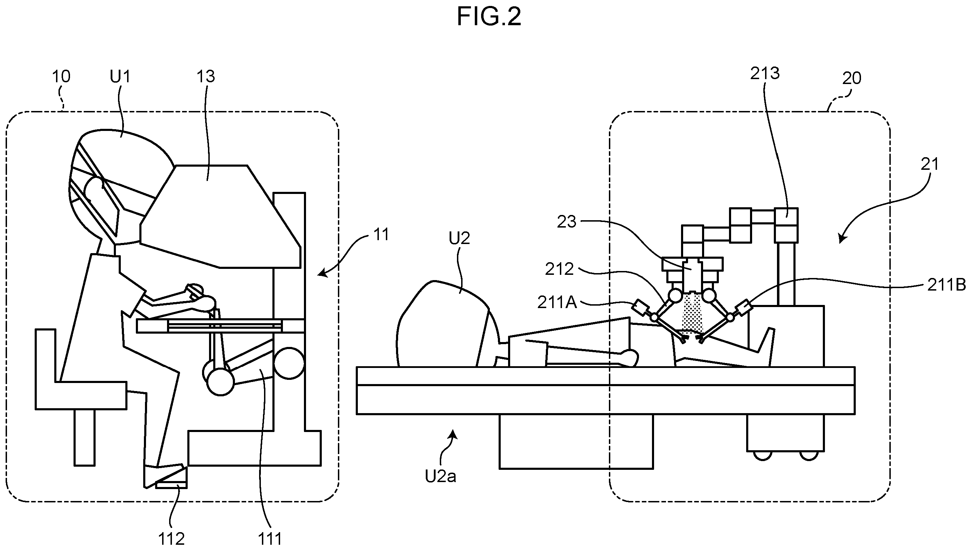

A configuration example of a master-slave system 1 according to a first embodiment will be described with reference to FIG. 1 and FIG. 2. FIG. 1 is a diagram illustrating a configuration of the master-slave system 1 according to the first embodiment. FIG. 2 is a diagram illustrating configuration examples of a master device 10 and a slave device 20 according to the first embodiment. The master-slave system 1 is a system using a master-slave type robot (master-slave robot).

As illustrated in FIG. 1, the master-slave system 1 includes the master device 10, the slave device 20, and a control device 30. The control device 30 is communicably connected to each of the master device 10 and the slave device 20. All of the devices transmit and receive various kinds of information to and from one another. The transmission and reception may be performed via various kinds of communication networks in one or both of a wireless manner and a wired manner, for example.

### Master device

The master device 10 includes a master robot 11, a robot control unit 12, a display unit 13, a communication unit 14, and a sensor unit 15. For example, the master device 10 has a function to control drive of the slave device 20, a function to provide information from the slave device 20 to a user, and the like.

The master robot 11 functions as an input device that allows a user, such as an operator, to perform remote operation on the slave device 20 on which a surgical tool, such as a forceps, is mounted. The master robot 11 has a configuration that is suitable for the user to perform the remote operation on the slave device 20. The master robot 11 operates based on a driving signal from the robot control unit 12.

The robot control unit 12 controls a position of the master robot 11 in accordance with a value of a position command that is designated by the user via the master robot 11 or the like. Examples of the control position include a position of a tip end portion (hand position) of the master robot 11. This control is performed in accordance with information (hand position command) that designates the position of the tip end portion. The hand position command may be generated in accordance with user operation or may be generated by the control device 30 at the time of bilateral control. The robot control unit 12 controls, for example, rotation (rotation speed, rotational angular velocity, torque, or the like) of a joint part such that the tip end portion of the master robot 11 is located at a position corresponding to the hand position command.

The display unit 13 displays various kinds of images. The display unit 13 mainly provides information on operation that is being performed on the slave device 20 to the user who is operating the master robot 11 based on image information that is acquired by the slave device 20. As the display unit 13, for example, an installation-type display, a Head Mounted Display (HMD) that is mounted on a head of the user, or the like may be used.

The communication unit 14 enables communication of various kinds of information with the control device 30. For example, the communication unit 14 transmits input information for the master robot 11 or sensor information that is obtained from the sensor unit 15 to the control device 30. Further, the communication unit 14 receives information (for example, control information, various kinds of information on the slave device 20, or the like) that is transmitted from the control device 30.

The sensor unit 15 detects a state of the master robot 11. Examples of the state to be detected include a torque reference value, an angle (joint angle), and angular velocity (joint angular velocity) of the joint part, and the information is used as the sensor information. The torque reference value substantially corresponds to a current value that is input to the master robot 11, and is detectable by the master device 10. The joint angle is acquired from, for example, an encoder in an actuator that is arranged in the joint part of the master robot 11. The joint angular velocity is acquired by differentiating the joint angle with respect to time. Other examples of the state to be detected include an acceleration reference value, a position, and velocity of the tip end portion, which are input to the master robot 11. The acceleration reference value is a value that is a base of the torque reference value as described above, and is detectable by the master device 10. The position of the tip end portion and the velocity of the tip end portion are detectable because they are identified from, for example, the joint angle, the joint angular velocity, or the like as described above.

### Slave device

The slave device 20 includes a slave robot 21, a robot control unit 22, an imaging unit 23, a communication unit 24, and a sensor unit 25. For example, the slave device 20 includes a mechanism that is driven by an actuator, such as a motor, and operates in accordance with drive control that is performed by the master device 10. Further, the slave device 20 has a function to provide, to the master device 10, a force and vibration of contact between a work object and a part of the slave device 20 that comes into contact with the object.

The slave robot 21 is a robot that is remotely operated by a user, such as an operator. For example, the slave robot 21 is an arm-type robot that has a multi-joint link structure, and includes an operation unit as an end effector that is mounted on a tip end of the robot. The slave robot 21 operates based on a driving signal from the robot control unit 22.

The robot control unit 22 controls a position of the slave robot 21 in accordance with a value of a position command that is designated by the user. Examples of the control position include a position of a tip end portion (hand position) of the slave robot 21. This control is performed in accordance with information (hand position command) that designates the position of the tip end portion. The hand position command may be generated in accordance with user operation or may be generated by the control device 30 at the time of bilateral control. The robot control unit 22 controls, for example, rotation (rotation speed, rotational angular velocity, torque, or the like) of a joint part such that the tip end portion of the slave robot 21 is located at a position corresponding to the hand position command.

The imaging unit 23 acquires, by imaging, an image of a work area of the object. For example, the imaging unit 23 includes a zoom mechanism and is able to change an imaging magnification (zoom magnification). The zoom magnification of the imaging unit 23 may be controlled by the control device 30. Meanwhile, the imaging unit 23 is arranged on the slave robot 21. The imaging unit 23 is supported by, for example, a robot arm other than a slave arm 212, and the robot control unit 22 controls a joint angle or the like of the robot arm. A position and a posture of the imaging unit 23 may be changed with this configuration. As the imaging unit 23, for example, an RGB camera, a stereo camera, or the like may be used.

The communication unit 24 enables communication of various kinds of information with the control device 30. For example, the communication unit 24 receives information (for example, control information, various kinds of information on the master device 10, or the like) that is transmitted from the control device 30. Further, the communication unit 24 transmits sensor information that is obtained from the sensor unit 25 to the control device 30.

The sensor unit 25 detects a state of the slave robot 21. Examples of the state to be detected include a torque reference value, an angle (joint angle), and angular velocity (joint angular velocity) of the joint part, and the information is used as the sensor information. The torque reference value substantially corresponds to a current value that is input to the slave robot 21, and is detectable by the slave device 20. The joint angle is acquired from, for example, an encoder in an actuator that is arranged in the joint part of the slave robot 21. The joint angular velocity is acquired by differentiating the joint angle with respect to time. Other examples of the state to be detected include an acceleration reference value, a position, and velocity of the tip end portion, which are input to the slave robot 21. The acceleration reference value is a value that is a base of the torque reference value as described above, and is detectable by the slave device 20. The position of the tip end portion and the velocity of the tip end portion are detectable because they are identified from, for example, the joint angle, the joint angular velocity, or the like as described above.

### Control device

The control device 30 includes a control unit 31, a storage unit 32, and a communication unit 33. The control unit 31 includes a drive control unit 311, a display control unit 312, an area setting unit 313, a determination unit 314, and a synchronous switching unit 315.

The drive control unit 311 controls each of units that are related to drive and that are included in the master-slave system 1. For example, the drive control unit 311 acquires the sensor information that is transmitted from the master device 10, and acquires a master position that is a position of the master robot 11 based on the acquired sensor information. Further, the drive control unit 311 controls a slave position that is a position of the slave robot 21 based on the acquired information on the master position or the like.

For example, the drive control unit 311 controls the master robot 11 and the slave robot 21 such that the position of the master robot 11 and the position of the slave robot 21 correspond to each other. Positional correspondence described herein indicates that positions of corresponding portions of the master robot 11 and the slave robot 21 have a correspondence relationship. For example, the master robot 11 and the slave robot 21 are controlled such that a position of the tip end portion of the master robot 11 and a position of the tip end portion of the slave robot 21 are located so as to have a correspondence relationship.

Further, the drive control unit 311 controls the master robot 11 and the slave robot 21 such that an external force of the master robot 11 and an external force of the slave robot 21 correspond to each other. Correspondence of the external forces described herein indicate that external forces of corresponding portions of the master robot 11 and the slave robot 21 have a correspondence relationship. For example, the master robot 11 and the slave robot 21 are controlled such that an external force of the tip end portion of the master robot 11 and an external force of the tip end portion of the slave robot 21 have a correspondence relationship.

The display control unit 312 mainly controls each of units that are related to a video and that are included in the master-slave system 1. For example, the display control unit 312 acquires image information that is transmitted from the slave device 20, and controls the display unit 13 of the master device 10 or the like based on the acquired image information. At this time, the display control unit 312 generates various kinds of images based on the image information and sends the various kinds of generated images to the display unit 13.

The area setting unit 313 sets an interference avoidance area of the slave robot 21. The interference avoidance area is, for example, an area in which the tip end portion of the slave robot 21 can avoid interference with the surrounding environment, such as a user or a bed. The interference avoidance area is set in advance. The interference avoidance area as described above will be described in detail later.

The determination unit 314 determines whether or not an operation interface (input device) of the master robot that is operated by a user is deviated from a predetermined operation area in operation of the master-slave robot. Specific operation of the determination unit 314 will be described later.

The synchronous switching unit 315, when the operation interface is deviated from the predetermined operation area, performs switching so as to disable synchronization between the slave robot and the master robot. Detailed operation of the synchronous switching unit 315 will be described later.

The storage unit 32 stores therein various kinds of information. For example, the storage unit 32 stores therein various kinds of information, such as control information, sensor information, and image information. The information as described above is, for example, information that is transmitted from one or both of the master device 10 and the slave device 20. Further, the storage unit 32 stores therein various kinds of information (for example, a program or the like) that are needed for a process that is performed by the control device 30.

The communication unit 33 enables communication of various kinds of information with the master device 10 and the slave device 20. For example, the communication unit 33 receives information (for example, various kinds of information on the master device 10, various kinds of information on the slave device 20, or the like) that is transmitted from the master device 10 and the slave device 20. Further, the communication unit 33 transmits various kinds of information (for example, control information, sensor information, image information, or the like) to the master device 10 and the slave device 20.

The master-slave system 1 as described above is used for, for example, operation as illustrated in FIG. 2. In the example illustrated in FIG. 2, an operator (doctor or the like) who performs operation is illustrated as a user U1, and a patient who is subjected to the operation is illustrated as a user U2. The user U2 lies on a bed U2a or the like, for example.

As illustrated in FIG. 2, the user U1 operates the master device 10 to remotely operate the slave device 20 to perform the operation on the user U2. Specifically, the master device 10 includes the master robot 11 that is operated by the user U1. The slave device 20 includes the slave robot 21 that is remotely operated by the user U1. Meanwhile, in the present embodiment, the master robot 11 and the slave robot 21 are medical robots, but may be industrial robots in an industrial field other than the medical field.

The master robot 11 has a configuration that is suitable for the user U1 to remotely operate the slave robot 21. The master robot 11 illustrated by example holds a master arm 111, the display unit 13, and the like. The master arm 111 is an arm that is operated by the user U1. Details of the operation (user operation) that is performed by the user U1 on the master robot 11 are transmitted, as control information (input information), from the master device 10 to the slave device 20 via the control device 30. Further, the master robot 11 includes, for example, a component that corresponds to the slave robot 21 so as to notify the user U1 of the state of the slave robot 21. The master robot 11 may include, for example, an arm that is similar to the slave robot 21. The user U1 is able to recognize the state of the slave robot 21 via the master robot 11. Meanwhile, a foot switch 112 may be arranged on a floor between hands of the master robot 11. When the foot switch 112 is pressed, a signal is transmitted from the master device 10 to the control device 30.

The master arm 111 is operated by, for example, both of a right hand and a left hand of the user U1. The user U1 places both arms or both elbows on a support table, and grips the master arm 111 by each of the right hand and the left hand. In this state, the user U1 operates the master arm 111 while viewing the display unit 13 that displays an operative field. The user U1 changes a position and orientation of the master arm 111 to remotely operate a position or orientation of a surgical tool that is mounted on the slave device 20 or remotely operate a grip operation of each of surgical tools, for example. The master arm 111 is able to convey, to the user U1, a feel when the surgical tool of the slave device 20 comes into contact with an affected area or the like of the patient.

The slave robot 21 includes, for example, an arm. The slave robot 21 illustrated by example includes a plurality of surgical tool units 211A and 211B, the slave arm 212, and a base arm 213. The slave robot 21 has, for example, six degrees of freedom with respect to a tip end position and a posture, but the number of the degrees of freedom of the robot is not specifically limited. Each of the surgical tool units 211A and 211B is held by the slave arm 212. In the example illustrated in FIG. 2, the two surgical tool units are provided. Each of the slave arm 212 and the base arm 213 includes, for example, a plurality of link parts and a plurality of joint parts. The base arm 213 holds, for example, the slave arm 212, the imaging unit 23, and the like. Meanwhile, examples of each of surgical tools of each of the surgical tool units 211A and 211B include a forceps, a tweezers, a scissors, an insufflation tube, an energy treatment tool, and a retractor.

Here, in the master device 10, an operation command for remotely operating the slave robot 21 is input via the master robot 11. The operation command includes, for example, pan operation and tilt operation on the slave arm 212 of the slave robot 21, rotation operation around a longitudinal axis (roll axis) of each of the surgical tool units 211A and 211B, operation on each of surgical tool tip of each of the surgical tool units 211A and 211B, and the like. The control device 30 generates a control command based on the received operation command, and transmits the control command to the slave device 20. The slave device 20 controls drive of the slave robot 21 to realize operation of the slave robot 21 in accordance with the received control command.

For example, in the master device 10, operation of the slave robot 21 or operation of the surgical tool (for example, yaw operation, pitch operation, open-close operation, or the like of a forceps) is designated via the master robot 11. For example, when the control device 30 receives an operation command that designates the yaw operation, the pitch operation, and the open-close operation of a forceps, the control device 30 calculates a rotation angle of each of motors of the slave robot 21 to realize operation of the target forceps, and generates an angular command for each of the motors. A control command that includes the angular command or the like is transmitted to the slave device 20.

### 1-1-1. Definition of slave-side operation area and slave-side safe area

Conventionally, there is a known method in which, when a user is not operating a master, the master is adjusted to a position and a posture of a slave. Therefore, an actuator that copes with positional deviation between the master and the slave is mounted on the master. When the actuator is mounted on the master, a size of the master increases. Further, there is a known method in which, with use of a master having an increased size, the master itself adjusts a position of the master to the position and the posture of the slave. In this case, with increase in the size of the master, a size of a system increases. Furthermore, in this method, the position and the posture of the slave may be different from the position and the posture at the time the master is operated, and therefore, operational independence of the user may be degraded even when positional alignment is performed.

Moreover, conventionally, operation of switching between synchronous and asynchronous, such as clutch operation, frequently occurs in a master-slave system, and therefore, it is expected that operation becomes more complicated. The master-slave system 1 according to one embodiment eliminates positional deviation between the master and the slave that frequently occurs in the master-slave system 1 and improve operability of the master-slave system 1.

Specifically, the master-slave system 1 determines whether or not the operation interface of the master robot 11 that is operated by the user is deviated from a predetermined operation area, and when the operation interface is deviated from the predetermined operation area, the master-slave system 1 performs switching so as to disable the synchronization between the slave robot and the master robot. After disabling the synchronization between the slave robot and the master robot, the master-slave system 1 moves the slave robot to a safe area at the side of the slave. When receiving operation for resynchronization, the master-slave system 1 performs positional alignment with respect to the slave robot that is located in a slave-side safe area, and starts to synchronize the master robot and the slave robot. With this configuration, with respect to the positional deviation that frequently occurs between the master and the slave, the master-slave system 1 according to one embodiment performs switching so as to disable the synchronization between the slave robot and the master robot when the operation interface is deviated from the predetermined operation area. When receiving operation for resynchronization, the master-slave system 1 performs positional alignment with respect to the slave robot that is located in the slave-side safe area, so that it is possible to improve and correct operability as compared to the conventional technology.

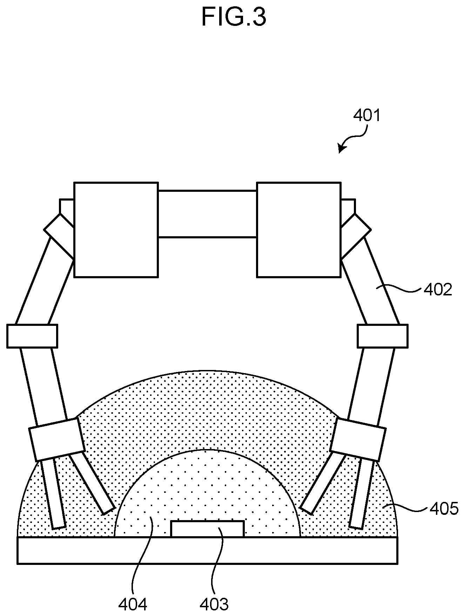

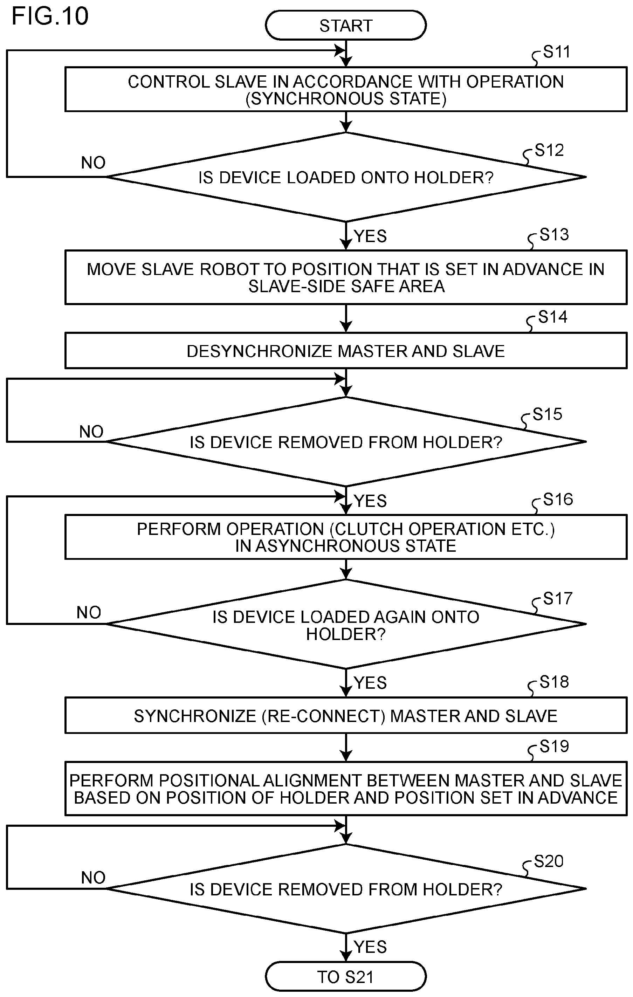

Firstly, a concept of a slave-side operation area and the slave-side safe area is important for explaining operation of the master-slave system 1 according to the first embodiment. Therefore, the slave-side operation area and the slave-side safe area will be described below. FIG. 3 is a side view for explaining a slave-side operation area 404 and a slave-side safe area 405. A master-side operation area and a master-side safe area will be described later with reference to FIG. 10.

In the master robot system, as illustrated in FIG. 3, the slave-side operation area 404 is provided in which a slave 401 performs operation on a target object 403 in a real space in accordance with user operation. The slave-side operation area 404 is a range in which the robot is able to contact with a target object 403. The slave-side operation area 404 is a range in which it is possible to touch a part in an assembly work, or a range in which it is possible to touch an affected area by mounting a surgical tool or the like on a surgical support robot. The slave-side operation area 404 includes a wheel or the like in a leg part, and the same applies to a robot that can autonomously move during operation of an operator.

A sphere whose diameter corresponds to a distance in which each of the left arm and the right arm of a robot arm 402 is able to come into contact with the target object 403 is adopted as the slave-side operation area 404. In this case, an outer area of the sphere in which the robot arm 402 is unable to come into contact with the target object 403 is adopted as the slave-side safe area 405 that is a safe distal area.

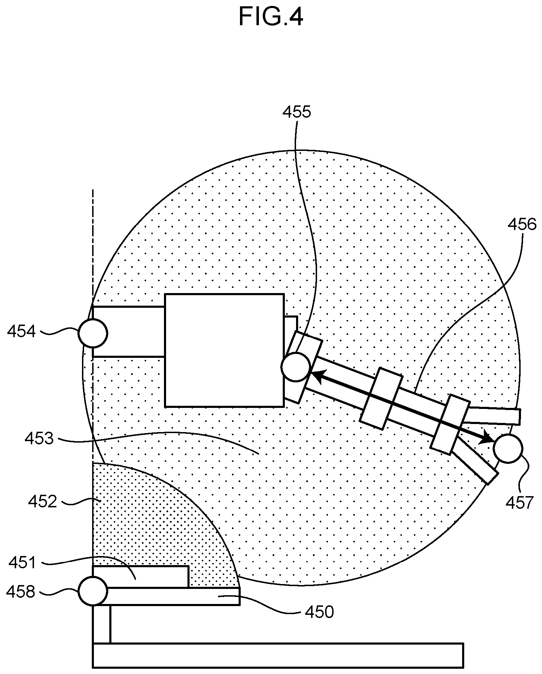

Definitions of a slave-side operation area and a slave-side safe area of a robot that has a single arm that is different from the arms illustrated in FIG. 3 will be described below. FIG. 4 is a diagram for explaining the definitions of a slave-side safe area in a robot model.

In FIG. 4, a slave-side operation area 452 is a semi-spherical area whose diameter corresponds to a distance from a center 458 of a stage 450 on which the target object 451 is set to an end of the stage 450. Here, the slave-side operation area 452 is a range in which the robot is able to come into contact with a target object as described above. The slave-side safe area is in a robot operating range in which the robot is unable to come into contact with the target object 451. An arm motion range 453 is a range in which the arm is operable in the robot model and is a slave-side safe area. It is assumed that the slave-side safe area is an area in which the robot is operable but is unable to touch the target object 451. In this case, an arm maximum motion range is a fixed value that is defined from information on the robot model, and the slave-side operation area 452 is a variable that varies depending on a size of the target object 451. Further, it is assumed that a person, an object, or the like does not intrude into the arm motion range 453 of the robot and there is no risk of contact.

In FIG. 4, a slave arm is mounted on a base center 454, and an end effector is mounted on a rotation axis 455 at an arm base. A distance between the rotation axis 455 at the arm base and an edge 457 of the end effector is an arm radius 456 of the arm.

### 1-1-2. Operation interface of master

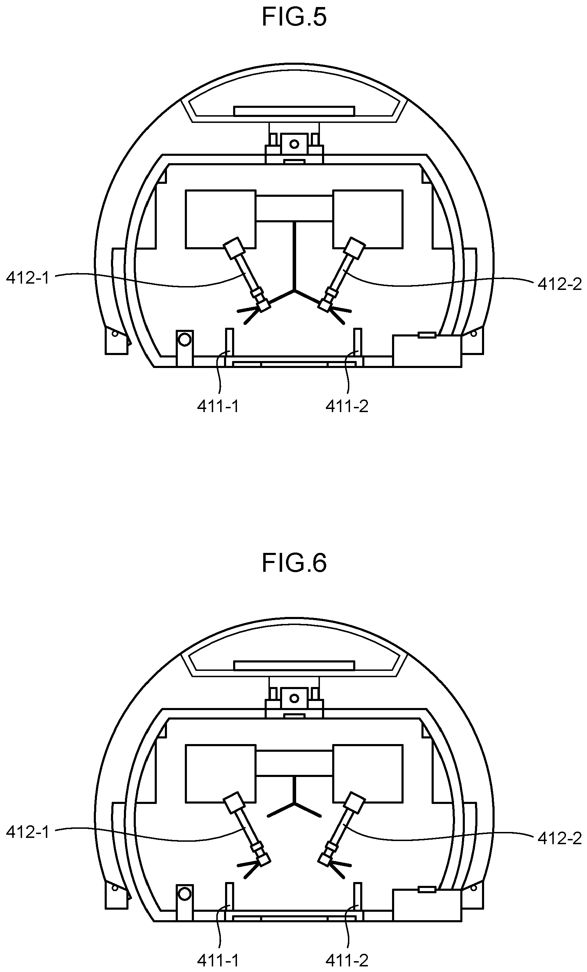

The operation interface of the master will be described below. The operation interface of the master includes a left-side master input device 412-1 and a right-side master input device 412-2. FIG. 5 is a diagram illustrating configuration examples of master input devices 412-1 and 412-2 on the left and right of the master according to the first embodiment. In the first embodiment, loading of the left-side master input device 412-1 onto a left-side holder 411-1 that is provided on a distal side on a console by a user is adopted as a switching trigger (hereinafter, also described as a "holder-type trigger") for switching between synchronous and asynchronous. Further, the loading of the right-side master input device 412-2 onto a right-side holder 411-2 that is provided on a distal side on the console by the user is adopted as a switching trigger for switching between synchronous and asynchronous.

As illustrated in FIG. 5, the operation interface of the master includes the left-side master input device 412-1 and the right-side master input device 412-2 for controlling the slave robot. Further, the left-side holder 411-1 for loading the left-side master input device 412-1 and the right-side holder 411-2 for loading the right-side master input device 412-2 are provided.

The operation interface of the master is a terminal for conveying input operation that is performed by the user to the slave, independently of whether or not an actuator is mounted. When the user inputs an operation command or a command to the operation interface of the master, the operation command is transmitted to the slave via the control unit 31.

The left-side holder 411-1 and the right-side holder 411-2 are installed at positions at which the slave is fixed distally. Grounding positions of the left-side holder 411-1 and the right-side holder 411-2 may be fixed values of positions corresponding to the slave-side safe area. Grounding positions of the left-side holder 411-1 and the right-side holder 411-2 may be locked in the positions corresponding to the slave-side safe area. Further, the positions of the left-side holder 411-1 and the right-side holder 411-2 may be manually moved by a different person, such as a user or a nurse, while viewing an image of the slave.

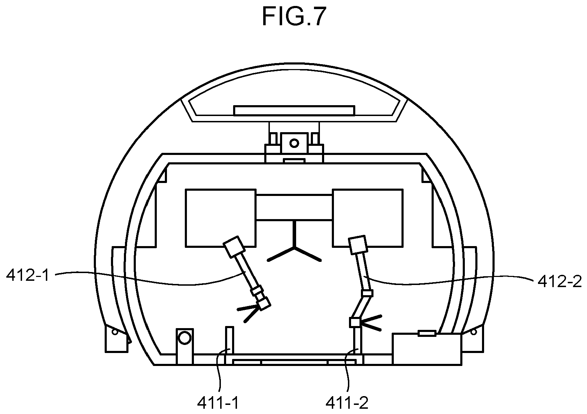

FIG. 6 is a diagram illustrating the left-side master input device 412-1 and the right-side master input device 412-2 of the master at the time of user operation according to the first embodiment. As illustrated in FIG. 6, at the time of the user operation, the user freely moves the right-side master input device 412-2 and the left-side master input device 412-1. FIG. 7 is a diagram illustrating the left-side master input device 412-1 and the right-side master input device 412-2 of the master at the time of trigger execution according to the first embodiment. At the time of the trigger execution, the left-side master input device 412-1 is mounted on the left-side holder 411-1 or the right-side master input device 412-2 is mounted on the right-side holder 411-2. FIG. 7 illustrates an example in which the right-side master input device 412-2 is mounted on the right-side holder 411-2.

### 1-1-3. Relationship between holder and master operation unit

Each of the left-side holder 411-1 and the right-side holder 411-2 includes, for example, a magnet and a contact sensor. The left-side holder 411-1 adsorbs, by a magnetic force, the left-side master input device 412-1 when the left-side master input device 412-1 approaches, and the determination unit 314 detects that the left-side master input device 412-1 is loaded onto the left-side holder 411-1. The right-side holder 411-2 adsorbs, by a magnetic force, the right-side master input device 412-2 when the right-side master input device 412-2 approaches, and the determination unit 314 detects that the right-side input device 412-2 is loaded onto the right-side holder 411-2. The synchronous switching unit 315 switches between the synchronous state and the asynchronous state while fixing the position and the posture, by adopting loading of the left-side master input device 412-1 onto the left-side holder 411-1 as a trigger. Further, the synchronous switching unit 315 switches between the synchronous state and the asynchronous state while fixing the position and the posture, by adopting loading of the right-side master input device 412-2 onto the right-side holder 411-2 as a trigger. Therefore, the slave is able to maintain the same position and the same posture in a safe zone before and after the switching.

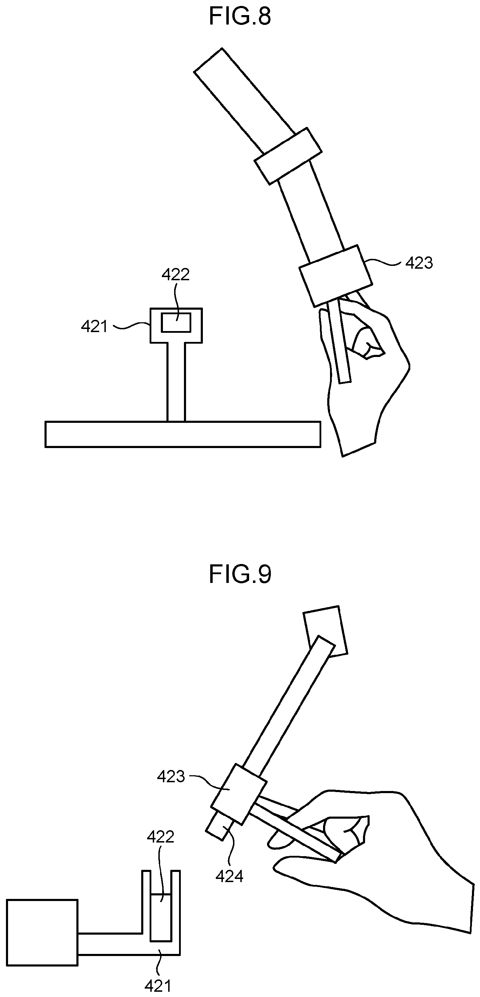

FIG. 8 is a top view of a holder 421 and a master operation unit 423 when the holder-type trigger according to the first embodiment is used. FIG. 9 is a side view of the holder 421 and the master operation unit 423 when the holder-type trigger according to the first embodiment is used.

As illustrated in FIG. 8 and FIG. 9, on the master console, the holder 421 for loading the master is mounted. The holder 421 has a hole for loading the master, and, inside the hole, a magnet 422 is provided for fixing the position and the posture when the master is loaded.

Further, the master operation unit 423 includes a magnet (not illustrated). Furthermore, a switch 424 is mounted in a portion in which the master operation unit 423 and the holder 421 come into contact with each other. The switch 424 detects loading of a part including the switch 424 of the master operation unit 423 of the master onto the holder 421. The synchronous switching unit 315 is able to switch between a synchronous state and an asynchronous state while adopting loading of the master onto the holder 421 as a trigger.

### 1-1-4. Operation

Operation of the master-slave system 1 according to the first embodiment will be described below. FIG. 10 is a flowchart for explaining the operation of the master-slave system 1 according to the first embodiment.

The control unit 31 receives operation in a synchronous state from the master, and controls the slave in accordance with the operation (Step S11). Subsequently, the determination unit 314 determines whether or not the input device of the master is loaded onto the holder (Step S12). In other words, the determination unit 314 determines whether or not the operation interface of the master robot that is operated by the user is deviated from the predetermined operation area.

Here, the "predetermined operation area" is the master-side operation area corresponding to an area in which the slave robot is able to touch a target object. The master-slave system 1 stores therein a correspondence relationship between a position of the slave-side operation area and a position of the master-side operation area. Further, the master-slave system 1 stores therein a correspondence relationship between a position of the slave-side safe area and a safe position of a master safe area.

For example, in the first embodiment, the master-slave system 1 determines "deviation from the predetermined operation area" based on whether or not "the operation interface is mounted on the holder". In other words, in the first embodiment, the "predetermined operation area" indicates an "area of the master-side operation area corresponding to an area in which the slave robot is able to touch the target object, except for a holder installation portion".

Specifically, in the first embodiment, for example, in FIG. 5 to FIG. 7, the "predetermined operation area" on the master side corresponds to an area other than the positions of the left-side holder 411-1 and the right-side holder 411-2. An outer side of the master-side "predetermined operation area" corresponds to the position of the left-side holder 411-1. When the left-side master input device 412-1 is mounted on the left-side holder 411-1, it is determined that the left-side master input device 412-1 is deviated from the predetermined operation area. Further, when the right-side master input device 412-2 is mounted on the right-side holder 411-2, it is determined that deviation from the predetermined operation area has occurred. At Step S12, when the input device of the master is not loaded onto the holder (No at Step S12), the process returns to Step S11.

At Step S12, when the input device of the master is loaded onto the holder (Yes at Step S12), the synchronous switching unit 315 moves the slave robot to a position that is set in advance in the slave-side safe area (Step S13).

Thereafter, the synchronous switching unit 315 desynchronizes the master and the slave (Step S14). In other words, when the operation interface is deviated from the predetermined operation area, the synchronous switching unit 315 performs switching so as to disable the synchronization between the slave robot and the master robot. The determination unit 314 determines whether or not the input device of the master is removed from the holder (Step S15). At Step S15, when the input device of the master is not removed from the holder (No at Step S15), the process returns to Step S11.

At Step S15, when the input device of the master is removed from the holder (Yes at Step S15), operation in an asynchronous state (clutch operation or the like) is performed (Step S16). Subsequently, the determination unit 314 determines whether or not the input device of the master is loaded again onto the holder (Step S17). At Step S17, when the input device of the master is not loaded again onto the holder (No at Step S17), the process returns to Step S16.

At Step S17, when the input device of the master is loaded again onto the holder (Yes at Step S17), the master and the slave are synchronized and re-connected (Step S18).

Thereafter, the synchronous switching unit 315 distally performs positional alignment between the master and the slave based on the position of the holder and a position that is set in advance (Step S19). In this case, to display state information on the slave to the user, the display unit 13 may give a notice of completion of the positional alignment of the slave by using a Graphical User Interface (GUI).

Subsequently, the determination unit 314 determines whether or not the input device of the master is removed from the holder (Step S20). At Step S20, when the input device of the master is not removed from the holder (No at Step S20), the process returns to Step S19.

At Step S20, when the input device of the master is removed from the holder (Yes at Step S20), the process returns to Step S11. With this configuration, the user is able to continue synchronous operation without performing positional alignment.

### 1-1-5. Example of calibration method

In the explanation as described above, the case has been described in which calibration of the safe area is not performed. However, it may be possible to perform calibration of the safe area.

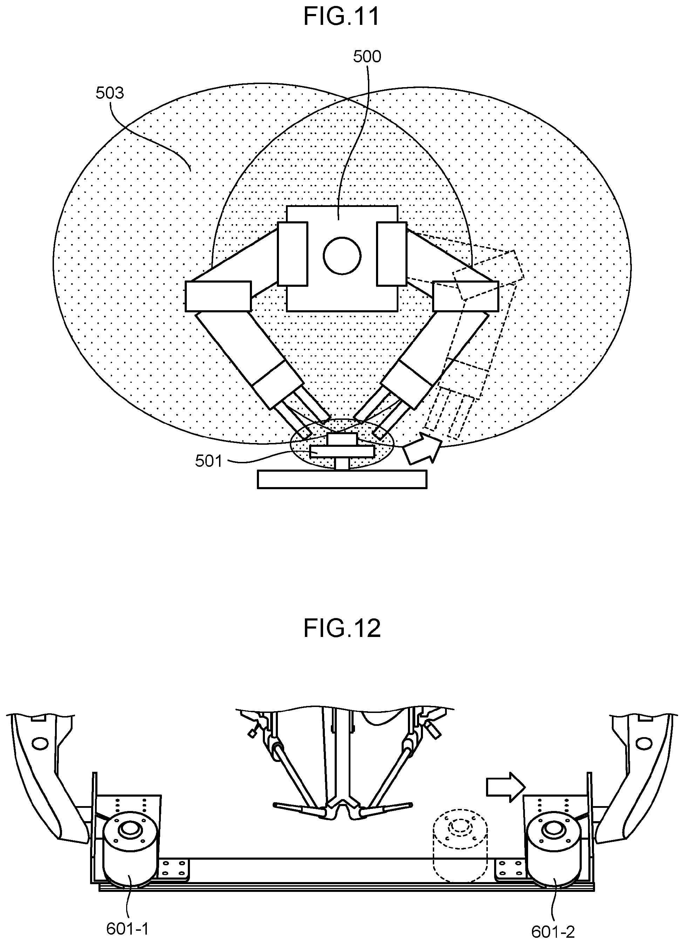

FIG. 11 is a diagram illustrating a slave-side operation area 501 and a slave-side safe area 503 of a robot 500. FIG. 12 is a diagram illustrating a state in which a right-side holder 601-2 of a master is automatically moved. FIG. 12 illustrates an example in which a position of the holder 601-2 is adjusted such that a position of the slave is located in the slave-side safe area 503.

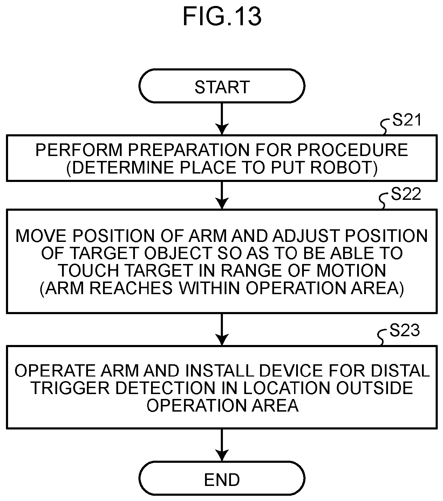

FIG. 13 is a diagram illustrating an example of calibration of the safe area according to the first embodiment. As illustrated in FIG. 13, preparation for a procedure is performed. Specifically, a place to put the robot is determined (Step S21).

Subsequently, the synchronous switching unit 315 moves a position of an arm, and adjusts a position of a target object so as to be able to touch the target in a range of motion (Step S22). Specifically, the synchronous switching unit 315 moves the arm until the arm reaches within the slave-side operation area 501.

Thereafter, the synchronous switching unit 315 operates the arm and installs a device for distal trigger detection (for example, the right-side holder 601-2 and a left-side holder 601-1) in a location outside the slave-side operation area 501 (Step S23).

### 1-1-6. Effect of first embodiment

According to the master-slave system 1 of the first embodiment, it is possible to eliminate positional deviation that frequently occurs between the master and the slave, and improve operability of the master-slave system 1.

### 1-2. Second embodiment

A master-slave system 1 according to a second embodiment will be described below. A configuration of the master-slave system 1 according to the second embodiment is the same as the example illustrated in FIG. 1 and FIG. 2, but, in the second embodiment, the master input device has a different configuration.

In the second embodiment, by adopting, as a trigger, approach or touch of a hand of an operator to a proximity sensor or a switch that is mounted on a distal side on the console, synchronous and asynchronous between the master and the slave are switched.

In the second embodiment, as one example, a positional alignment method in which a position and a posture are not fixed distally based on a user interface using a switch will be described. A master input device to be used has a similar configuration to the configuration explained in the first embodiment, and therefore, details will be omitted.

In the second embodiment, the master-slave system 1 determines whether or not "deviation from the predetermined operation area" has occurred based on position-posture information on the operation interface without using a holder. For example, the master-slave system 1 determines that "deviation from the predetermined operation area" has occurred based on whether or not the operation interface has approached an arbitrary target. As one example, the master-slave system 1 determines that "deviation from the predetermined operation area" has occurred when approach within a predetermined distance from the switch or the proximity sensor that is installed at the side of the master occurs. In this case, the "predetermined operation area" indicates "an area except for an installation portion of the switch or except for a sensing distance range of the proximity sensor in a master-side operation area corresponding to an area in which the slave robot is able to touch the target object".

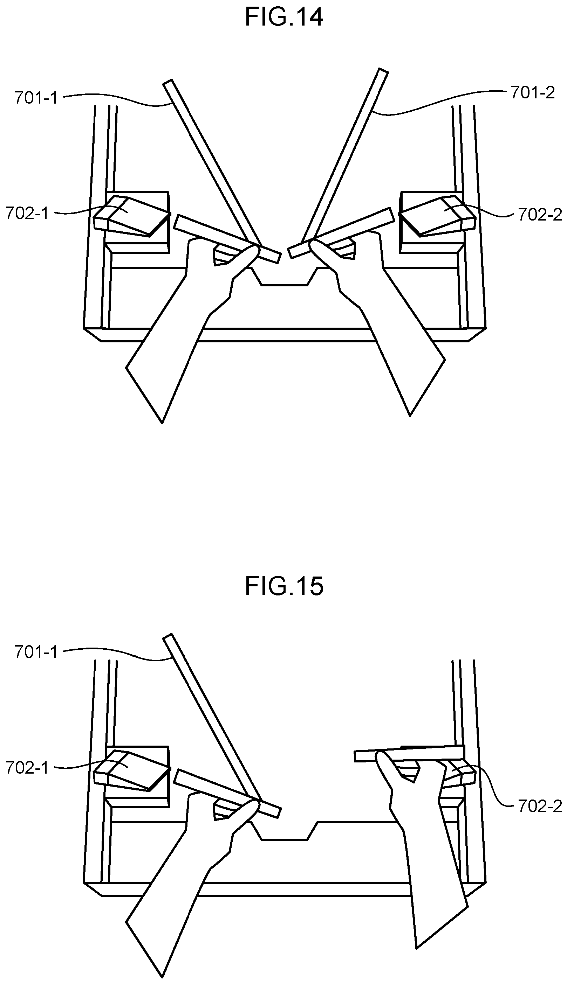

FIG. 14 is a diagram illustrating configuration examples of a left-side master input device 701-1 and a right-side master input device 701-2 of the master-slave system 1 according to the second embodiment. FIG. 14 illustrates a state in which a user is operating the master. FIG. 15 is a diagram illustrating a state in which the right-side master input device 701-2 of the master-slave system 1 according to the second embodiment executes a trigger. FIG. 15 illustrates a state in which a right hand of the user presses a right switch 702-2 while holding the left-side master input device 701-1 and the right-side master input device 701-2.

Firstly, as a configuration of an interface, as illustrated in FIG. 14, a left switch 702-1 that moves the slave to the safe area (distal) is mounted. Further, the right switch 702-2 that moves the slave to a right-side safe area (distal). Meanwhile, it may be possible to mount a proximity sensor instead of the left switch 702-1 and the right switch 702-2. The left switch 702-1 and the right switch 702-2 are mounted "outside the predetermined operation area" on the master side.

The user presses the left switch 702-1 or the right switch 702-2 (or the proximity sensor) while holding the left-side master input device 701-1 and the right-side master input device 701-2. With this configuration, the determination unit 314 of the master-slave system 1 is able to detect the trigger, and the synchronous switching unit 315 is able to switch between the synchronous state and the asynchronous state between the master and the slave.

When the proximity sensor is mounted instead of the switch 702-1 and the switch 702-2, the user brings the left-side master input device 701-1 or the right-side master input device 701-2 close to the proximity sensor. Accordingly, the determination unit 314 of the master-slave system 1 is able to detect the trigger, and the synchronous switching unit 315 is able to switch between the synchronous state and asynchronous state.

Meanwhile, detection that the user is holding the left-side master input device 701-1 or the right-side master input device 701-2 may be performed by a sensor (not illustrated) that is included in the left-side master input device 701-1 or the right-side master input device 701-2. Further, pressing of the switch 702-1 and the switch 702-2 may be allowed on condition that the sensor detects that the user is holding the left-side master input device 701-1 and the right-side master input device 701-2. Furthermore, when the sensor of the left-side master input device 701-1 detects that the user is not holding the left-side master input device 701-1 and when the switch 702-1 is pressed, the master-slave system 1 may output a warning message. Moreover, when the sensor of the right-side master input device 701-2 detects that the user is not holding the right-side master input device 701-2 and when the switch 702-2 is pressed, the master-slave system 1 may output a warning message.

Furthermore, when the left-side master input device 701-1 and the right-side master input device 701-2 have specific postures (for example, perpendicular case), the master-slave system 1 may perform calibration similarly to the first embodiment. Moreover, in this case, the master-slave system 1 may output, to the user, a message indicating that calibration is needed.

According to the second embodiment, as compared to the first embodiment, operation of placing the left-side master input device 701-1 and the right-side master input device 701-2 at fixed positions on the holder is not needed, so that it is satisfactory to touch the switch 702-1 or the switch 702-2 without being bothered by searching for the holder or independently of the postures, so that intuitive operation is improved.

### 1-2-1. Operation

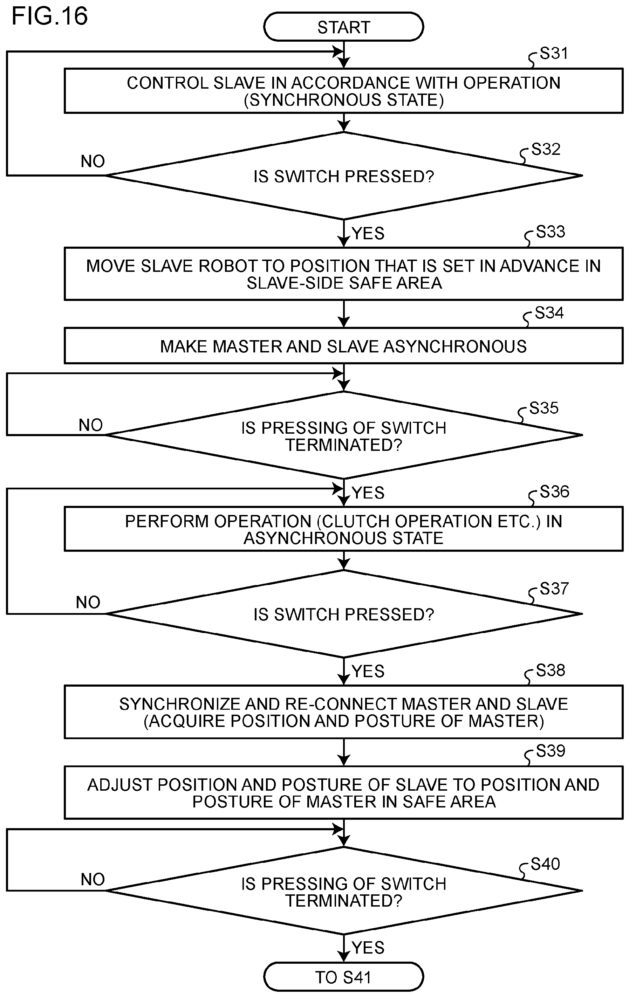

Operation of the master-slave system 1 according to the second embodiment will be described below. FIG. 16 is a flowchart for explaining the operation of the master-slave system 1 according to the second embodiment.

The control unit 31 receives operation in a synchronous state from the master, and controls the slave in accordance with the operation (Step S31). Subsequently, the determination unit 314 determines whether or not the switch is pressed (Step S32). In other words, the determination unit 314 determines whether or not the operation interface of the master robot that is operated by the user is deviated from the predetermined operation area. At Step S32, when the switch is not pressed (No at Step S32), the process returns to Step S31.

In the second embodiment, for example, in FIG. 14 and FIG. 15, the "predetermined operation area" on the master side corresponds to an area other than the position of the left switch 702-1 or the right switch 702-2. Deviation from the "predetermined operation area" on the master side corresponds to pressing of the switch, and corresponds to, for example, pressing of the left switch 702-1 or the right switch 702-2. In other words, when the left switch 702-1 or the right switch 702-2 is pressed, it is determined that the left-side master input device 701-1 or the right-side master input device 701-2 is deviated from the predetermined operation area.

At Step S32, when the switch is pressed (Yes at Step S32), the synchronous switching unit 315 moves the slave robot to the position that is set in advance in the slave-side safe area (Step S33). Meanwhile, pressing of the switch is performed such that the user presses the switch while holding the left-side master input device 701-1 and the right-side master input device 701-2.

In the second embodiment, the user executes the trigger by pressing the switch, which is installed on a distal side of the master, while moving the master during operation in a state in which the master and the slave are synchronized, and switches between the synchronous state and the synchronous state.

Thereafter, the synchronous switching unit 315 desynchronizes the master and the slave (Step S34), and the determination unit 314 determines whether or not pressing of the switch is terminated (Step S35). At Step S35, when it is determined that pressing of the switch is not terminated (No at Step S35), the process returns to Step S35.

At Step S35, when pressing of the switch is terminated (Yes at Step S35), operation in the asynchronous state (clutch operation or the like) is performed (Step S36). In the second embodiment, when synchronization between the master and the slave is disabled, the slave is stopped in a safe distal area that is deviated from the operation area and therefore is located in a safe state, so that the operator is able to perform arbitrary operation in the asynchronous state.

Subsequently, the determination unit 314 determines whether or not the switch is pressed (Step S37). At Step S37, when the switch is not pressed (No at Step S37), the process returns to Step S36.

At Step S37, when the switch is pressed (Yes at Step S37), the master and the slave are synchronized and reconnected (Step S38).

Meanwhile, when the proximity sensor is used instead of the switch, the proximity sensor is arranged outside the "predetermined operation area" on the master side. Deviation from the "predetermined operation area" on the master side corresponds to tapping of a hand of the user holding the master input device or the master input device so as to be detected by the proximity sensor. When the hand of the user holding the master input device or the master input device is detected by the proximity sensor, it is determined that deviation from the predetermined operation area has occurred.

At this time, as compared to the holder type of the first embodiment, in the second embodiment, the position and the posture of the left-side master input device 701-1 or the right-side master input device 701-2 are not fixed and unstable. Therefore, the position and the posture of the switch observed when the user presses the switch for the first time (hereinafter, also described as "master position and master posture in a synchronous state") are different from the position and the posture observed when the trigger is executed again by the switch (hereinafter, also described as "master position and master posture in an asynchronous state"). Therefore, the master-slave system 1 needs to acquire the position and the posture of the master at the time of transition from the asynchronous state to the synchronous state.

At Step S38, the synchronous switching unit 315 acquires the position and the posture of the master in the asynchronous state. The synchronous switching unit 315 calculates positional deviation that has occurred in the asynchronous state by comparing the acquired position and the acquired posture of the master and the position and the posture of the stopped slave. After calculating the positional deviation, the synchronous switching unit 315 performs positional alignment by moving the position and the posture of the slave so as to eliminate the deviation. The position and the posture of the master is acquired by using, for example, a gyro sensor, a position sensor, or the like.

Thereafter, the synchronous switching unit 315 adjusts the position and the posture of the slave to the position and the posture of the master in the safe area (distal) (Step S39). In this case, to display the state information on the slave to the user, the display unit 13 may give a notice indicating completion of the positional alignment of the slave by using a GUI.

Furthermore, the left-side master input device 701-1 or the right-side master input device 701-2 is caused to vibrate. The vibration of the master input device 701-1 or the right-side master input device 701-2 may give a feedback, to the user, that a robot of the slave arrives at the slave-side safe area. Various kinds of feedback methods may be adopted. For example, when the robot of the slave arrives at the slave-side safe area, it may be possible to output sound from the left-side master input device 701-1 or the right-side master input device 701-2. Moreover, with approach of the robot of the slave to the slave-side safe area, it may be possible to increase the vibration or the sound that is emitted from the left-side master input device 701-1 or the right-side master input device 701-2. For example, the control device 30 calculates a distance between the position of the left-side master input device 701-1 or the right-side master input device 701-2 of the slave and the position of the slave-side safe area, and outputs magnitude of the vibration or volume of the sound from the left-side master input device 701-1 or the right-side master input device 701-2 in accordance with the calculated distance. With this configuration, the user is able to check a timing at which clutch is performed.

Specifically, according to the second embodiment, by pressing the switch on the master console again, synchronization between the positions and the postures between the master and the slave is started in the safe area that is located distally.

Subsequently, the determination unit 314 determines whether or not pressing of the switch is terminated (Step S40). At Step S40, when pressing of the switch is not terminated (No at Step S40), the process returns to Step S40.

At Step S40, when pressing of the switch is terminated (Yes at Step S40), the process returns to Step S31. With this configuration, the user is able to continue synchronous operation without performing positional alignment.

### 1-2-2. Effects of second embodiment

Therefore, according to the master-slave system 1 of the second embodiment, while positional alignment is being performed, the user need not perform special operation in accordance with the positional alignment, so that the operation is not interrupted. Further, because the operation is not interrupted, the user does not lose the sense of control over the operation.

Furthermore, according to the master-slave system 1 of the second embodiment, it is possible to use the switch when the asynchronous state is returned to the synchronous state, so that operation performed by the user is not interrupted. Moreover, unlike the holder, the posture is not fixed with use of the switch, so that it is possible to start operation in an arbitrary posture as an initial position.

### 2. Other embodiments

Processes according to each of the embodiments as described above may be implemented in various different modes other than the embodiments as described above.

Further, of the processes described in the embodiments, all or part of a process described as being performed automatically may also be performed manually. Alternatively, all or part of a process described as being performed manually may also be performed automatically by known methods. In addition, the processing procedures, specific names, and information including various kinds of data and parameters illustrated in the above-described document and drawings may be arbitrarily changed unless otherwise specified. For example, various kinds of information illustrated in each of the drawings are not limited to the information illustrated in the drawings.

Furthermore, the components of the apparatuses illustrated in the drawings are functionally conceptual and do not necessarily have to be physically configured in the manner illustrated in the drawings. In other words, specific forms of distribution and integration of the apparatuses are not limited to those illustrated in the drawings, and all or part of the apparatuses may be functionally or physically distributed or integrated in arbitrary units depending on various loads or use conditions.

Moreover, the embodiments and the modifications as described above may be combined appropriately as long as processing contents do not conflict with each other.

Furthermore, the effects described in the present specification are illustrated by example, and do not pose any limitation; therefore, other effects may be achieved.

### 3. Effects

As described above, a master-slave system (the master-slave system 1 in one embodiment) according to the present disclosure includes a determination unit (the determination unit 314 in one embodiment) that determines, in operation of a master robot and a slave robot, whether or not an operation interface of the master robot that is operated by a user is deviated from a predetermined operation area, and a synchronous switching unit (the synchronous switching unit 315 in one embodiment) that performs switching so as to disable synchronization between the slave robot and the master robot when the operation interface (the master input devices 412-1 and 412-2 in one embodiment) deviates from the predetermined operation area.

In this manner, when the operation interface is deviated from the predetermined operation area, the master-slave system according to the present disclosure is able to disable the synchronization between the slave robot and the master robot, so that it is possible to eliminate positional deviation between the master and the slave during an asynchronous state. In addition, the master-slave system of the present disclosure need not cause the master robot to actively perform positional alignment, so that there are no limitations on installation of an actuator in the master-slave system. As a result, it is possible to reduce a design cost and a size of the master-slave system of the present disclosure.

Furthermore, when the operation interface is deviated from the predetermined operation area, the synchronous switching unit moves the slave robot to a slave-side safe area and performs switching so as to disable the synchronization.

In this manner, the master-slave system according to the present disclosure moves the slave robot to the slave-side safe area, so that it is possible to safely perform positional alignment between the master robot and the slave robot.

Moreover, when the operation interface of the master robot is mounted on a holder that is installed outside the operation area, the determination unit determines that the operation interface of the master robot is deviated from the predetermined operation area. When it is determined that the operation interface of the master robot is deviated from the predetermined operation area, the synchronous switching unit performs control such that the slave robot is moved to a position that is set in advance in the slave-side safe area.

In this manner, in the master-slave system according to the present disclosure, when the operation interface is mounted on the holder, a position and a posture of the operation interface are uniquely determined. Therefore, it is possible to perform positional alignment between the master robot and the slave robot without a need of a process for acquiring position-posture information on the operation interface. Furthermore, the master robot need not actively perform positional alignment, so that operation performed by the user is not interrupted. Moreover, the positional alignment is performed at a safe position, so that it is possible to safely perform the positional alignment.

Furthermore, when receiving, from the user, operation for resynchronization, the synchronous switching unit performs positional alignment between the master robot and the slave robot based on a position of the holder and a position that is set in advance in the slave-side safe area, and starts synchronization between the master robot and the slave robot.

In this manner, the master-slave system according to the present disclosure uses the position of the holder for the positional alignment between the master robot and the slave robot, so that it is possible to set the position of the holder as a fixed value and simplify calculation for the positional alignment.

Moreover, the determination unit acquires position-posture information on the operation interface of the master robot, and determines whether or not the operation interface of the master robot is deviated from the predetermined operation area based on the position-posture information. When it is determined that the operation interface of the master robot is deviated from the predetermined operation area, the synchronous switching unit performs control such that the slave robot moves to the slave-side safe area.

In this manner, the master-slave system according to the present disclosure performs positional alignment based on the position-posture information on the operation interface of the master robot, so that it is not needed to cause the master robot to actively perform positional alignment. Therefore, operation performed by the user is not interrupted. Further, the slave robot is moved to the slave-side safe area, so that it is possible to safely perform positional alignment between the master robot and the slave robot.

Furthermore, when receiving, from the user, operation for resynchronization, the synchronous switching unit performs positional alignment with respect to a slave robot that is located in the safe area based on the position-posture information on the operation interface of the master robot, and starts synchronization between the master robot and the slave robot.

In this manner, the master-slave system according to the present disclosure is able to perform resynchronization in the safe area.

Moreover, the master-slave system includes a display unit that displays completion of positional alignment of a position of the slave robot with respect to a position of the master robot.

In this manner, in the master-slave system according to the present disclosure, the user is able to confirm completion of the positional alignment and continue operation.

Furthermore, in the master-slave system, the operation interface of the master robot includes a first magnet, and the holder includes, inside thereof, a second magnet that attracts to the first magnet.

In this manner, the master-slave system according to the present disclosure is able to improve connectivity between the operation interface and the holder by the first magnet and the second magnet.

Moreover, in the master-slave system, the holder includes a switch in a portion in which the operation interface is mounted, and when the switch is pressed, the determination unit determines that the operation interface of the master robot is deviated from the predetermined operation area.

In this manner, the master-slave system according to the present disclosure is able to infallibly determine that the operation interface of the master robot is deviated from the predetermined operation area.

Furthermore, the master robot includes a switch that is installed outside the operation area, and, when the switch is pressed, the synchronous switching unit determines that the operation interface is deviated from the predetermined operation area and desynchronizes the master robot and the slave robot.

In this manner, with use of the switch, the master-slave system according to the present disclosure allows the user to desynchronize the master robot and the slave robot in flexible positions and flexible postures.

Moreover, after desynchronizing the master robot and the slave robot, when the switch is pressed, the synchronous switching unit synchronizes the master robot and the slave robot.

In this manner, the master-slave system according to the present disclosure is able to use the switch even when restoring from asynchronous to synchronous, so that operation performed by the user is not interrupted. Further, unlike the holder, the posture is not fixed with use of the switch, so that it is possible to start operation in an arbitrary posture as an initial position.

Furthermore, when the slave robot arrives at the slave-side safe area, the determination unit gives a feedback to the user.

In this manner, the master-slave system according to the present disclosure allows the user to recognize safety.

Moreover, the determination unit increases one of vibration and sound that is emitted from the operation interface with approach of the slave robot to the slave-side safe area.

In this manner, the master-slave system according to the present disclosure allows the user to strongly recognizes safety.

Furthermore, the determination unit performs calibration on the slave robot for determining the slave-side safe area.

In this manner, in the master-slave system according to the present disclosure, even when the position of the operation interface is specific (for example, vertical case), by performing the calibration, the determination unit is able to determine whether or not the operation interface is deviated from the predetermined operation area.

Moreover, when receiving operation for resynchronization, the synchronous switching unit acquires position-posture information on the operation interface of the master robot.

In this manner, in the master-slave system according to the present disclosure, when a switch or the like is used to determine deviation from a predetermined area, it is possible to use the position-posture information that is acquired for positional alignment.

### 4. Hardware configuration

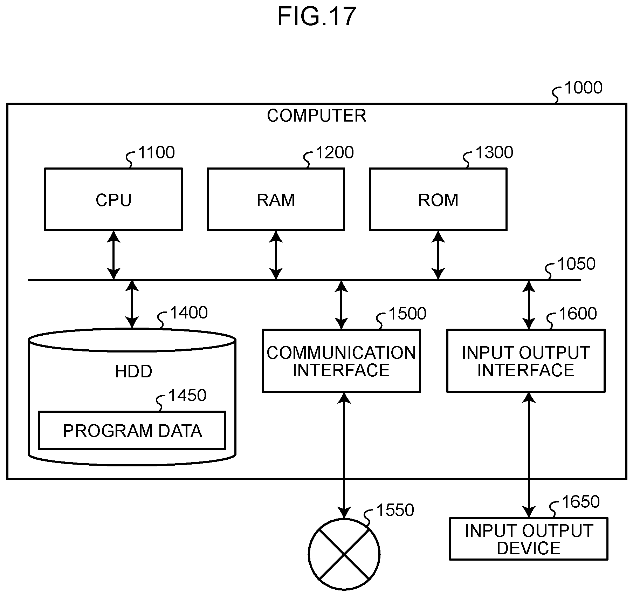

FIG. 17 is a hardware configuration diagram illustrating an example of a computer that implements an arithmetic device of the master-slave system 1 as an information processing apparatus according to the first embodiment and the second embodiment.

A computer 1000 includes a CPU 1100, a RAM 1200, a Read Only Memory (ROM) 1300, a Hard Disk Drive (HDD) 1400, a communication interface 1500, and an input output interface 1600. All of the units of the computer 1000 are connected to one another via a bus 1050.

The CPU 1100 operates based on a program that is stored in the ROM 1300 or the HDD 1400 and controls each of the units. For example, the CPU 1100 loads a program that is stored in the ROM 1300 or the HDD 1400 onto the RAM 1200, and performs processes corresponding to various kinds of programs.

The ROM 1300 stores therein a boot program, such as a Basic Input Output System (BIOS), that is executed by the CPU 1100 at the time of activation of the computer 1000, a program that is dependent on hardware of the computer 1000, or the like.

The HDD 1400 is a computer readable recording medium that non-transitory records therein a program that is executed by the CPU 1100, data that is used by the program, and the like. Specifically, the HDD 1400 is a recording medium that stores therein an application program according to the present disclosure that is one example of program data 1450.

The communication interface 1500 is an interface that allows the computer 1000 to connect to an external network 1550 (for example, the Internet). For example, the CPU 1100 receives data from a different device or transmit data generated by the CPU 1100 to a different device, via the communication interface 1500.

The input output interface 1600 is an interface for connecting an input output device 1650 and the computer 1000. For example, the CPU 1100 receives data from an input device, such as a keyboard or a mouse, via the input output interface 1600. Further, the CPU 1100 transmits data to an output device, such as a display, a speaker, or a printer, via the input output interface 1600. Furthermore, the input output interface 1600 may function as a media interface that reads a program or the like that is recorded in a predetermined recording medium (medium). Examples of the medium include an optical recording medium, such as a Digital Versatile Disc (DVD) or a Phase change rewritable disk (PD), a magneto-optical recording medium, such as a Magneto-Optical disk (MO), a tape medium, a magnetic recording medium, and a semiconductor memory.

Meanwhile, the CPU 1100 reads the program data 1450 from the HDD 1400 and executes the program data; however, as another example, it may be possible to acquire the above-described programs from a different apparatus via the external network 1550.

Thus, preferred embodiments of the present disclosure have been described in detail above with reference to the accompanying drawings, the technical scope of the present disclosure is not limited to the examples as described above. It is obvious that a person skilled in the technical field of the present disclosure may conceive various alternations and modifications within the scope of the appended claims, and it should be understood that they will naturally come under the technical scope of the present disclosure.

Furthermore, the effects described in this specification are merely illustrative or exemplified effects, and are not limitative. That is, with or in the place of the above effects, the technology according to the present disclosure may achieve other effects that are clear to those skilled in the art from the description of this specification.

### 5. Notes

Meanwhile, the present technology may also be configured as below.
(1) A master-slave system comprising:
   a determination unit that determines, in operation of a master robot and a slave robot, whether or not an operation interface of the master robot that is operated by a user is deviated from a predetermined operation area; and
   a synchronous switching unit that performs switching so as to disable synchronization between the slave robot and the master robot when the operation interface is deviated from the predetermined operation area.
(2) The master-slave system according to (1), wherein when the operation interface is deviated from the predetermined operation area, the synchronous switching unit moves the slave robot to a slave-side safe area and performs switching so as to disable the synchronization.
(3) The master-slave system according to (2), wherein
   when the operation interface of the master robot is mounted on a holder that is installed outside the operation area, the determination unit determines that the operation interface of the master robot is deviated from the predetermined operation area, and
   when it is determined that the operation interface of the master robot is deviated from the predetermined operation area, the synchronous switching unit performs control such that the slave robot is moved to a position that is set in advance in the slave-side safe area.
(4) The master-slave system according to (3), wherein when receiving, from the user, operation for resynchronization, the synchronous switching unit performs positional alignment between the master robot and the slave robot based on a position of the holder and a position that is set in advance in the slave-side safe area, and starts synchronization between the master robot and the slave robot.
(5) The master-slave system according to any one of (2) to (4), wherein
   the determination unit acquires position-posture information on the operation interface of the master robot, and determines whether or not the operation interface of the master robot is deviated from the predetermined operation area based on the position-posture information, and
   when it is determined that the operation interface of the master robot is deviated from the predetermined operation area, the synchronous switching unit performs control such that the slave robot moves to the slave-side safe area.
(6) The master-slave system according to (5), wherein
   when receiving, from the user, operation for resynchronization, the synchronous switching unit performs positional alignment with respect to a slave robot that is located in the slave-side safe area based on the position-posture information on the operation interface of the master robot, and starts synchronization between the master robot and the slave robot.
(7) The master-slave system according to any one of (4) to (6), further comprising:
   a display unit that displays completion of positional alignment of a position of the slave robot with respect to a position of the master robot.
(8) The master-slave system according to any one of (3) to (7), wherein
   the operation interface of the master robot includes a first magnet, and
   the holder includes, inside thereof, a second magnet that attracts to the first magnet.
(9) The master-slave system according to any one of (3) to (8), wherein
   the holder includes a switch in a portion in which the operation interface is mounted, and
   when the switch is pressed, the determination unit determines that the operation interface of the master robot is deviated from the predetermined operation area.
(10) The master-slave system according to any one of (6) to (9), further comprising:
   a display unit that displays completion of positional alignment of a position of the slave robot with respect to a position of the master robot.
(11) The master-slave system according to (5) or (6), wherein
   the master robot includes a switch that is installed outside the predetermined operation area, and
   when the switch is pressed, the synchronous switching unit determines that the operation interface is deviated from the predetermined operation area and desynchronizes the master robot and the slave robot.
(12) The master-slave system according to (11), wherein after desynchronizing the master robot and the slave robot, when the switch is pressed, the synchronous switching unit synchronizes the master robot and the slave robot.
(13) The master-slave system according to (2), wherein when the slave robot arrives at the slave-side safe area, the determination unit gives a feedback, from the operation interface, to the user.
(14) The master-slave system according to (13), wherein the determination unit increases one of vibration and sound that is emitted from the operation interface with approach of the slave robot to the slave-side safe area.
(15) The master-slave system according to any one of (2) to (14), wherein the determination unit performs calibration on the slave robot for determining the slave-side safe area.
(16) The master-slave system according to any one of (6) to (15), wherein when receiving operation for resynchronization, the synchronous switching unit acquires position-posture information on the operation interface of the master robot.
(17) A master-slave system comprising:
   a control device;
   a master device that is connected to the control device; and
   a slave device that is connected to the control device, wherein
   the slave device includes
      a slave robot that performs operation on a patient,
   the control device includes
      a determination unit that determines whether or not an operation interface of a master robot that is operated by a user is deviated from the predetermined operation area; and
   a synchronous switching unit that performs switching so as to disable the synchronization between the slave robot and the master robot when the operation interface is deviated from the predetermined operation area,
   the master device includes
      the operation interface of the master robot that is operated by the user; and
      a holder that is installed outside the operation area, and
   when the operation interface is mounted on the holder that is installed outside the operation area, the determination unit of the control device determines that the operation interface is deviated from the predetermined operation area.
(18) A synchronous switching control method implemented by a computer, the synchronous switching control method comprising:
   determining, in operation of a master robot and a slave robot, whether or not an operation interface of the master robot that is operated by a user is deviated from a predetermined operation area; and
   performing switching so as to disable the synchronization between the slave robot and the master robot when the operation interface is deviated from the predetermined operation area.
(19) A program that causes a computer to function as:
   a determination unit that determines, in operation of a master robot and a slave robot, whether or not an operation interface of the master robot that is operated by a user is deviated from a predetermined operation area; and
   a synchronous switching unit that performs switching so as to disable the synchronization between the slave robot and the master robot when the operation interface is deviated from the predetermined operation area.

### Reference Signs List

1 master-slave system
314 determination unit
315 synchronous switching unit
404, 452 slave-side operation area
405 slave-side safe area
411-1, 411-2 holder
412-1, 412-2 master input device
422 magnet
423 master operation unit
424 switch

## Claims

1. A master-slave system comprising:
a determination unit that determines, in operation of a master robot and a slave robot, whether or not an operation interface of the master robot that is operated by a user is deviated from a predetermined operation area; and
a synchronous switching unit that performs switching so as to disable synchronization between the slave robot and the master robot when the operation interface is deviated from the predetermined operation area.

2. The master-slave system according to claim 1, wherein when the operation interface is deviated from the predetermined operation area, the synchronous switching unit moves the slave robot to a slave-side safe area and performs switching so as to disable the synchronization.

3. The master-slave system according to claim 2, wherein
when the operation interface of the master robot is mounted on a holder that is installed outside the operation area, the determination unit determines that the operation interface of the master robot is deviated from the predetermined operation area, and
when it is determined that the operation interface of the master robot is deviated from the predetermined operation area, the synchronous switching unit performs control such that the slave robot is moved to a position that is set in advance in the slave-side safe area.

4. The master-slave system according to claim 3, wherein when receiving, from the user, operation for resynchronization, the synchronous switching unit performs positional alignment between the master robot and the slave robot based on a position of the holder and a position that is set in advance in the slave-side safe area, and starts synchronization between the master robot and the slave robot.

5. The master-slave system according to claim 2, wherein
the determination unit acquires position-posture information on the operation interface of the master robot, and determines whether or not the operation interface of the master robot is deviated from the predetermined operation area based on the position-posture information, and
when it is determined that the operation interface of the master robot is deviated from the predetermined operation area, the synchronous switching unit performs control such that the slave robot moves to the slave-side safe area.

6. The master-slave system according to claim 5, wherein
when receiving, from the user, operation for resynchronization, the synchronous switching unit performs positional alignment with respect to a slave robot that is located in the slave-side safe area based on the position-posture information on the operation interface of the master robot, and starts synchronization between the master robot and the slave robot.

7. The master-slave system according to claim 4, further comprising:
a display unit that displays completion of positional alignment of a position of the slave robot with respect to a position of the master robot.

8. The master-slave system according to claim 3, wherein
the operation interface of the master robot includes a first magnet, and
the holder includes, inside thereof, a second magnet that attracts to the first magnet.

9. The master-slave system according to claim 3, wherein
the holder includes a switch in a portion in which the operation interface is mounted, and
when the switch is pressed, the determination unit determines that the operation interface of the master robot is deviated from the predetermined operation area.

10. The master-slave system according to claim 6, further comprising:
a display unit that displays completion of positional alignment of a position of the slave robot with respect to a position of the master robot.

11. The master-slave system according to claim 6, wherein
the master robot includes a switch that is installed outside the predetermined operation area, and
when the switch is pressed, the synchronous switching unit determines that the operation interface is deviated from the predetermined operation area and desynchronizes the master robot and the slave robot.

12. The master-slave system according to claim 11, wherein after desynchronizing the master robot and the slave robot, when the switch is pressed, the synchronous switching unit synchronizes the master robot and the slave robot.

13. The master-slave system according to claim 2, wherein when the slave robot arrives at the slave-side safe area, the determination unit gives a feedback, from the operation interface, to the user.

14. The master-slave system according to claim 13, wherein the determination unit increases one of vibration and sound that is emitted from the operation interface with approach of the slave robot to the slave-side safe area.

15. The master-slave system according to claim 2, wherein the determination unit performs calibration on the slave robot for determining the slave-side safe area.

16. The master-slave system according to claim 6, wherein when receiving operation for resynchronization, the synchronous switching unit acquires position-posture information on the operation interface of the master robot.

17. A master-slave system comprising:
a control device;
a master device that is connected to the control device; and
a slave device that is connected to the control device, wherein
the slave device includes
a slave robot that performs operation on a patient,
the control device includes
a determination unit that determines whether or not an operation interface of a master robot that is operated by a user is deviated from the predetermined operation area; and
a synchronous switching unit that performs switching so as to disable the synchronization between the slave robot and the master robot when the operation interface is deviated from the predetermined operation area,
the master device includes
the operation interface of the master robot that is operated by the user; and
a holder that is installed outside the operation area, and
when the operation interface is mounted on the holder that is installed outside the operation area, the determination unit of the control device determines that the operation interface is deviated from the predetermined operation area.

18. A synchronous switching control method implemented by a computer, the synchronous switching control method comprising:
determining, in operation of a master robot and a slave robot, whether or not an operation interface of the master robot that is operated by a user is deviated from a predetermined operation area; and
performing switching so as to disable the synchronization between the slave robot and the master robot when the operation interface is deviated from the predetermined operation area.

19. A program that causes a computer to function as:
a determination unit that determines, in operation of a master robot and a slave robot, whether or not an operation interface of the master robot that is operated by a user is deviated from a predetermined operation area; and
a synchronous switching unit that performs switching so as to disable the synchronization between the slave robot and the master robot when the operation interface is deviated from the predetermined operation area.
